# EUROPEAN PATENT APPLICATION

(11) **EP 4 417 236 A1**
(43) Date of publication of application: **21.08.2024**
(21) Application number: 24156851.8
(22) Date of filing: 09.02.2024
(51) Int. Cl.: A61M 16/00

(54) **UNCONSCIOUS MASK REMOVAL DETECTION AND ADAPTIVE ALERTING BY USING RESPIRATORY AND BEHAVIOURAL SIGNALS**

(30) Priority: 14.02.2023 EP 23156554
(71) Applicant: Koninklijke Philips N.V., 5656 AG Eindhoven (NL)
(72) Inventor: SHIM, Hee, Reen, 5656 AG Eindhoven (NL); DE RUYTER, Boris, Emmanuel, Rachmund, 5656 AG Eindhoven (NL); KRANS, Jan, Martijn, 5656 AG Eindhoven (NL)
(74) Representative: Philips Intellectual Property & Standards

(57) **Abstract**

A system (10) and method for unconscious mask removal detection and adaptive alerting in delivering a flow of gas via a mask (18) to a user's respiratory system comprises an unconscious mask removal detection module (30), a wake-up alert detection module (36), and an adaptive alert module (38). The unconscious mask removal detection module (30) outputs an indication of unconscious removal mask-off or other than removal. The wake-up alert decision module (36) outputs a decision whether to not activate or activate a wake-up alert based on (i) the unconscious mask removal detection module output and (ii) a cost-benefit analysis. The adaptive alert module (38) (i) determines alert settings for the wake-up alert based on (a) behavioral data (58) and (b) historical data (60), and (ii) sends the alert settings to an actuator (28). The actuator (28) actuates the wake-up alert in response to the determined wake-up alert settings to wake-up the user (20) so that the user can put the unconsciously removed mask on again.

## Description

### BACKGROUND

The present embodiments relate generally to continuous positive airway pressure (CPAP) systems and more particularly, to a system with unconscious mask removal detection and adaptive alerting by using respiratory and behavioral signals and a method thereof.

Unconsciously removing a CPAP mask during sleep is the one of the most common issues with CPAP. Since CPAP adherence is determined by the frequency and usage duration of the CPAP mask, solving this issue could help therapy adherence.

Continuous positive airway pressure (CPAP) therapy uses a CPAP machine to help a patient with obstructive sleep apnea (OSA) breathe more easily during sleep. A CPAP machine pumps air through a mask a patient wears at night.

One of the most commonly reported issues from CPAP users is unconscious mask removal: CPAP masks users remove their mask while they are sleeping without realizing they did so. Since unconscious mask removal during sleep could lead to poor compliance and decrease CPAP therapy effect, addressing this unconscious mask removal issue would be beneficial for therapy adherence. Unconscious mask removal is associated with nasal congestion and mouth leaks and might be caused by discomfort from the mask or inappropriate air pressure setting.

Although unconscious mask removal is a well-known issue, there is no optimal solution yet to reduce its negative impact. Patients who report unconscious mask removal are often recommended to change their mask type or adjust the air pressure settings to be more comfortable. However, these solutions have a limited positive impact on CPAP adherence: research found that CPAP adherence did not differ between different mask types. Also, it lacks supporting evidence that adaptive air pressure settings improve CPAP adherence. Therefore, it would be desirable to have a technical solution for reducing the negative impact of unconscious mask removal.

While patients with this issue are typically recommended to change their masks or air pressure settings, there is no optimal solution yet to reduce the negative impact of unconscious mask removal during sleep.

In other words, unconscious mask removal is an obstacle that blocks a patient from adhering to CPAP therapy. If this unconscious mask removal issue is unsolved, patients could miss therapy opportunities which could be detrimental for their healthcare. Existing solutions are either advising patients to get used to their CPAP masks and air pressure or providing additional features, such as bi-level PAP and adaptive air pressure setting. However, studies have shown that these approaches lead to no differences in CPAP compliance. In addition, none of the existing technical interventions reduces the negative impact of unconscious mask removal. Thus, it would be desirable to provide a system to directly address the negative impact of unconscious mask removal for reducing the loss of treatment opportunities during the night.

PCT patent application WO 2015/196255 A1 discloses a device for detecting a sealing condition between a patient interface and a patient, and adjusting the patient interface to maintain the patient interface in sealing contact with the patient. The patient interface may include a sealing structure to form a seal on the patient, and a positioning structure to secure the sealing structure to the patient.

Accordingly, an improved method and apparatus for overcoming the problems in the art is desired.

### SUMMARY

In accordance with one aspect, an automatic system is configured to detect sleep-onset (or one or more sleep stages) and mask status (whether removed or not) with an adaptive alert module. Upon detection of mask removal during sleep, the subject or patient is alerted to put his or her mask back on. As such, the negative impact of unconscious mask removal on therapy adherence is advantageously reduced. In accordance with another aspect, to reduce the negative impact of unconscious mask removal on CPAP therapy adherence, the system is configured to 1) detect unconscious mask removal; 2) decide a wake-up alert; and 3) adjust personalized alert settings, as will be discussed further herein.

According to one embodiment, a system for unconscious mask removal detection and adaptive alerting in delivering a flow of gas via a mask worn by the user to an airway of a user respiratory system comprises an unconscious mask removal detection module, a wake-up alert detection module, and an adaptive alert module. The unconscious mask removal detection module is configured to detect an unconscious mask removal, wherein the mask removal detection module outputs an indication of unconscious removal mask-off or other than unconscious removal mask-off, wherein the indication of other than unconscious removal mask-off includes an indication of (i) mask-on or (ii) intentional removal mask-off. The wake-up alert decision module is configured to output a decision whether to not activate or activate a wake-up alert based on (i) the output of the unconscious mask removal detection module and (ii) a cost-benefit analysis regarding whether to wake up the user having a positive or negative result based on at least a user treatment goal and user treatment history data. The adaptive alert module is configured to (i) determine alert signal settings for the wake-up alert based on (a) behavioral data of a previous response or previous responses to an alert by the user and (b) historical data of previous alert settings and a response to each previous alert setting by the user, and (ii) send the determined alert signal settings to an actuator for the actuator to actuate the wake-up alert in response to the determined wake-up alert signal settings to wake-up the user so that the user can put the unconsciously removed mask on again, wherein the adaptive alert module is operable in response to a decision output of the wake-up alert decision module to activate the wake-up alarm.

In one embodiment, the unconscious mask removal detection module further includes a sleep-onset detection module and a mask removal detection module, wherein the sleep-onset detection module is configured to process first sensor signals to detect a sleep-onset or identify sleep stages of the user, and wherein the mask removal detection module is configured to process second sensor signals to detect a status of the mask, whether the mask is removed or not. The first sensor signals comprise respiratory signals, actigraphy signals, or a combination of both. The second sensor signals comprise airflow sensor signals, pressure sensor signals, in-mask sensor signals, IR sensor signals, or a combination thereof.

In another embodiment, the sleep-onset detection module outputs (i) a non-sleep status in response to not detecting the sleep-onset or a predetermined identified sleep stage, and (ii) an in-sleep status in response to detecting the sleep-onset or another predetermined identified sleep state. In addition, the mask removal detection module outputs (i) a mask-off status in response to detecting that the user removed a CPAP mask, and (ii) a mask-on status in response to not detecting that the user removed the CPAP mask.

In a further embodiment, the unconscious mask removal detection module outputs an indication of (i) no removal decision, (ii) an intentional removal decision, and/or (iii) an unconscious removal decision. The no removal decision is output in response to (a) the sleep-onset detection module outputting the in-sleep status and the mask removal detection module outputting the mask-on status or (b) the sleep-onset detection module outputting the non-sleep status and the mask removal detection module outputting the mask-on status. The intentional removal decision is output in response to the sleep-onset detection module outputting the non-sleep status and the mask removal detection module outputting the mask-off status. The unconscious removal decision is output in response to the sleep-onset detection module outputting the in-sleep status and the mask removal detection module outputting the mask-off status.

In another embodiment, the wake-up alert decision module is further configured to compute the cost-benefit analysis result. A negative cost benefit analysis result is indicative of an expected cost of waking the user being higher that an expected benefit of extending the duration of wearing the mask after alerting the user, wherein the treatment goal has already been achieved or is above a given threshold of being achieved, and the expected benefit of waking up the user is less than a given low benefit threshold (i.e., marginal). A positive cost benefit analysis result is indicative of the expected benefit being higher that the expected cost of waking the user, wherein the treatment goal has not been achieved or is below a given threshold of being achieved and the unconscious mask removal is detected at the beginning of the sleep-onset, and the expected benefit of waking up the user is more than a given high benefit threshold (i.e., large). The wake-up alert decision module outputs the decision to (i) not activate the wake-up alert in response to a negative cost-benefit analysis result and the indication of unconscious removal mask-off or (ii) activate the wake-up alert in response to a positive cost-benefit analysis result and the indication of unconscious removal mask-off.

According to another embodiment, he alert signal settings include one or more of a vibration intensity, vibration frequency, or other vibration characteristic (e.g., duty-cycle, modulation, etc.). The behavioral data can include data relating to the user's prior response or responses to a given alert that includes one or more of ignoring the given alert, activating a snooze function for the given alert, or other recordable action with respect to the given alert. The historical data can include data relating to a most effective alert setting or settings, compared to a lesser effective alert setting or settings, from a previous night or previous nights to wake up the user.

In another embodiment, the adaptive alert module is further configured to update one or more of the behavioral data and historical data after each session of wake-up alert activation. The actuator may include one or more of a smartwatch, a wearable device with a vibration function, a mobile device, an alarm clock, a positive airway pressure (PAP) base station, or other vibration device (e.g., bed, mattress, or sleeping pad vibration device). The adaptive alert module may further be configured to adjust one or more setting of a continuous positive airway pressure (CPAP) system, used in delivering the flow of gas via the mask to the airway of the user respiratory system, based on user behavior relating to a threshold number of unconscious mask removal detections, wherein the threshold number is adapted with respect to a given CPAP implementation and user CPAP treatment history.

According to another embodiment, a continuous positive airway pressure (CPAP) system for delivering a flow of gas to an airway of a patient respiratory system featuring unconscious mask removal detection with adaptive alerting comprises a gas flow generator, at least one sensor, and a system for unconscious mask removal detection and adaptive alerting. The gas flow generator can be configured to generate the flow of gas, wherein the gas flow generator is further configured to communicate the flow of gas to a patient circuit that includes a mask. The at least one sensor can be configured to generate output signals related to at least one characteristic associated with at least one of respiratory, actigraphy, airflow, humidity, pressure, temperature, in-mask sensor signals, or any combination thereof. The system for unconscious mask removal detection and adaptive alerting is configured for deciding whether or not to activate the wake-up alarm, for determining alert signal settings for the wake-up alert, and for sending the determined alert signal settings to an actuator for the actuator to actuate the wake-up alert in response to the determined wake-up alert signal settings to wake-up the user so that the user can put the unconsciously removed mask on again.

According to one embodiment, a method for unconscious mask removal detection and adaptive alerting in a system for delivering a flow of gas via a mask worn by a user to an airway of a user respiratory system can comprise detecting unconscious mask removal, deciding whether to activate or not a wake-up alert, and determining alert signal settings. Detecting the unconscious mask removal can include detecting, via the unconscious mask removal module, and outputting an indication of unconscious removal mask-off or other than unconscious removal mask-off. The indication of other than unconscious removal mask-off may include an indication of mask-on or intentional removal mask-off. Deciding whether to activate or not the wake-up alert can include deciding, via the wake-up alert decision module, whether to not activate or activate a wake-up alert based on (i) the output of the unconscious mask removal detection module and (ii) a cost-benefit analysis regarding whether to wake up the user having a positive or negative result based on at least a user treatment goal and user treatment history data. Lastly, determining alert signal settings can include determining, via the adaptive alert module, alert signal settings for the wake-up alert based on (a) behavioral data of a previous response or previous responses to an alert by the user and (b) historical data of previous alert settings and a response to each previous alert setting by the user. The method further includes sending, via the adaptive alert module, the determined alert signal settings to the actuator for the actuator to actuate the wake-up alert in response to the determined wake-up alert signal settings to wake-up the user so that the user can put the unconsciously removed mask on again. The adaptive alert module is operable in response to a decision output of the wake-up alert decision module to activate the wake-up alarm.

In another embodiment, the method can include wherein detecting, via the unconscious mask removal detection module, further includes detecting, via a sleep-onset detection module and a mask removal detection module. The sleep-onset detection module is configured to process first sensor signals to detect a sleep-onset or identify sleep stages of the user. The mask removal detection module is configured to process second sensor signals to detect a status of a mask, whether the mask is removed or not. The first sensor signals can comprise respiratory signals, actigraphy signals, or a combination of both. The second sensor signals can comprise airflow sensor signals, pressure sensor signals, in-mask sensor signals, IR sensor signals (e.g., to detect a distance between a PAP mask and a user's forehead), or a combination thereof.

According to yet another embodiment, the method includes wherein deciding, via the wake-up alert decision module, further comprises computing the cost-benefit analysis result. A negative cost benefit analysis result is indicative of an expected cost being higher that an expected benefit, wherein the treatment goal has already been achieved or is above a given threshold of being achieved, and the expected benefit of waking up the user (to have the user put the mask on again) is less than a given low benefit threshold (i.e., marginal). A positive cost benefit analysis result is indicative of the expected benefit being higher that the expected cost, wherein the treatment goal has not been achieved or is below the given threshold of being achieved and the unconscious mask removal is detected at the beginning of the sleep-onset, and the expected benefit of waking up the user (to have the user put the mask on again) is more than a given high benefit threshold (i.e., large).

Advantages and benefits will become apparent to those of ordinary skill in the art upon reading and understanding the following detailed description.

### BRIEF DESCRIPTION OF THE DRAWINGS

The embodiments of the present disclosure may take form in various components and arrangements of components, and in various steps and arrangements of steps. Accordingly, the drawings are for purposes of illustrating the various embodiments and are not to be construed as limiting the embodiments. In the drawing Figs, like reference numerals refer to like elements. In addition, it is to be noted that the Figs may not be drawn to scale.
Fig. 1 is a block diagram view of a system for unconscious mask removal detection and adaptive alerting in delivering a flow of gas via a mask worn by the user to an airway of a user respiratory system according to an embodiment of the present disclosure;
Fig. 2 is a block diagram view of the controller of Fig. 1 in further detail according to an embodiment of the present disclosure; and
Fig. 3 is a flow diagram view of a method for unconscious mask removal detection and adaptive alerting in a system for delivering a flow of gas via a mask worn by a user to an airway of a user respiratory system according to an embodiment of the present disclosure.

### DETAILED DESCRIPTION

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

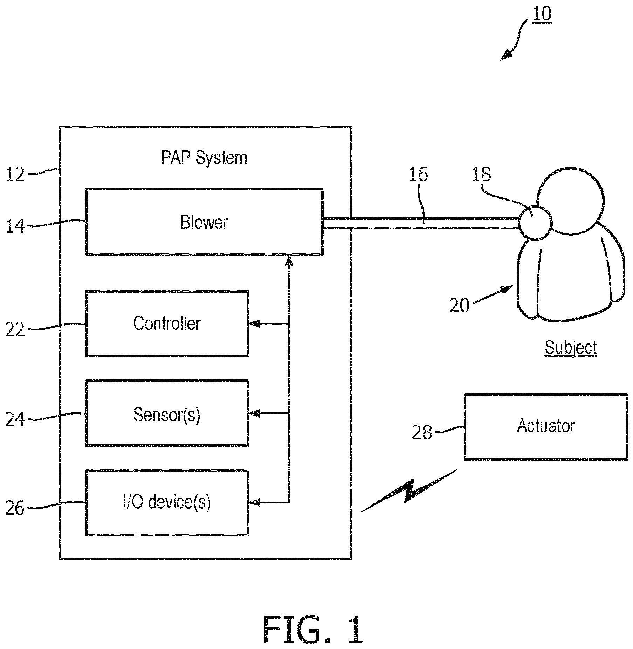

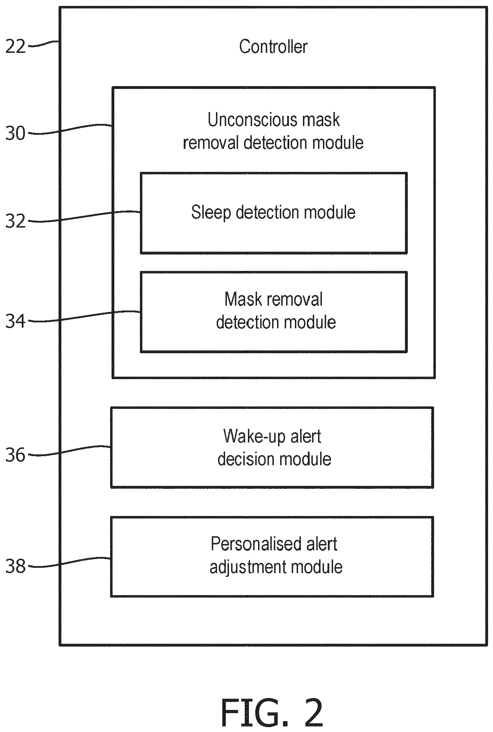

With reference to Fig. 1, there is shown a system 10 for delivering a flow of gas to an airway of a patient respiratory system featuring unconscious mask removal detection with adaptive alerting. The system 10 comprises a positive airway pressure system 12 having a gas flow generator or blower 14 configured to generate the flow of gas. The gas flow generator 14 is configured to communicate the flow of gas to a patient circuit 16 that includes a mask 18 to a patient or subject 20. The system further includes a controller 22 configured to implement one or more modules, as will be explained with reference to Fig. 2. In one embodiment, controller 22 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given PAP system implementation and/or application.

Controller 22 can further comprise one or more various modules configured to implement a given functionality as discussed herein. For example, the modules may comprise one or more of an integrated circuit, discrete analog or digital circuit components, hardware, software, firmware, or any combination thereof, for performing various functions as discussed herein, further according to the requirements of a given unconscious mask removal detection with adaptive alerting implementation and/or application. In addition, one or more of the modules may further comprise various combinations of one or more of the various modules as discussed herein. Furthermore, it is understood that the described modules may be computer program modules which are rendered in a non-transitory computer-readable medium.

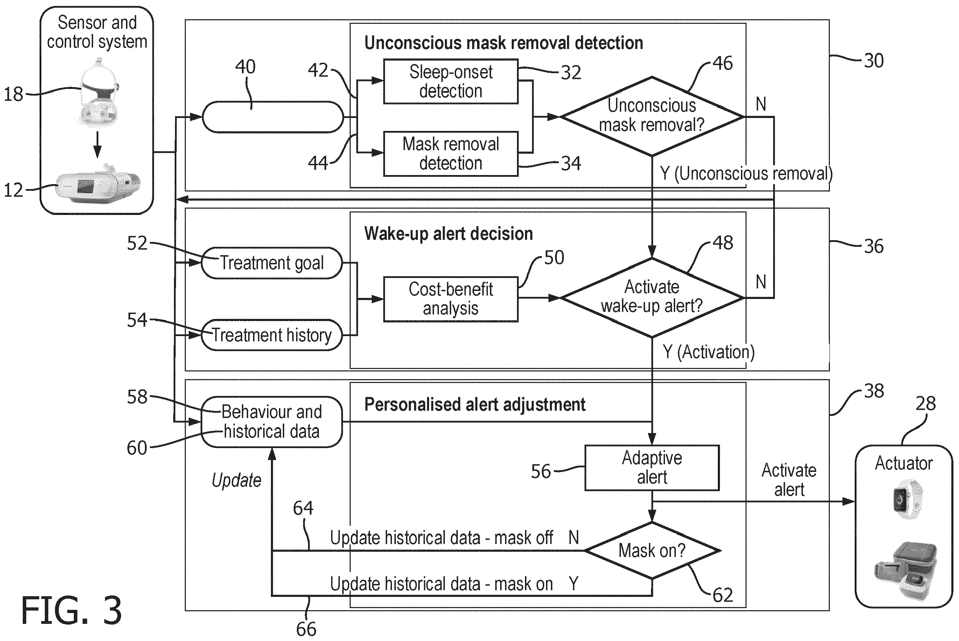

One or more sensor 24 (or sensors) are provided and configured to generate output signals (e.g., sensor signals 40, Fig. 3) related to at least one characteristic associated with at least one of respiratory, actigraphy, airflow, humidity, pressure, temperature, in-mask sensor signals, or any combination thereof. As will be discussed further herein, first sensor signals (e.g., signals 42, Fig 3) can comprise respiratory signals, actigraphy signals, or a combination of both. Second sensor signals (e.g., signals 44, Fig. 3) can comprise airflow sensor signals, pressure sensor signals, in-mask sensor signals, IR sensor signals (e.g., to detect a distance between a PAP mask and a user's forehead), or a combination thereof.

One or more input/output (I/O) device(s), collectively indicated by reference numeral 26, which is representative of any number of suitable I/O devices for inputting or outputting one or more of an audible, visual, and/or tactile input or output, according to the requirements of a given PAP system implementation featuring unconscious mask removal detection with adaptive alerting, further as discussed herein. For example, input/output device(s) 26 can comprise one or more of an input/output device, a user interface, tactile output device, touch screen, optical display, microphone, keypad, keyboard, pointing device, image capture device, video camera, audio output device, and any combination thereof, according to the requirements of the given PAP system implementation featuring unconscious mask removal detection with adaptive alerting. In another embodiment, input/output device 26 may include a mobile phone app configured for inputting or outputting one or more of an audible, visual, and/or tactile input or output, according to the requirements of a given PAP system implementation featuring unconscious mask removal detection with adaptive alerting.

System 10 further includes at least one actuator 28. As will be discussed further herein, the system decides whether or not to activate a wake-up alarm, determines alert signal settings for the wake-up alert and sends the determined alert signal settings to the at least one actuator 28. The actuator 28 then actuates the wake-up alert in response to the determined wake-up alert signal settings to wake-up the user 20 so that the user can put the unconsciously removed mask 18 on again. According to one embodiment, the actuator 28 includes one or more of a smartwatch, a wearable device with a vibration function, a mobile device, an alarm clock, a positive airway pressure (PAP) base station, or other vibration device (e.g., bed, mattress, or sleeping pad vibration device).

With reference now to Figs 2 and 3, in one embodiment, controller 22 comprises an unconscious mask removal detection module 30 configured to detect an unconscious mask removal, wherein the mask removal detection module outputs an indication of unconscious removal mask-off or other than unconscious removal mask-off, as indicated by decision block 46. The indication of other than unconscious removal mask-off includes an indication of (i) mask-on or (ii) intentional removal mask-off.

The unconscious mask removal detection module 30 includes a sleep-onset detection module 32 and a mask removal detection module 34. The sleep-onset detection module 32 is configured to process first sensor signals 42 to detect a sleep-onset or identify sleep stages of the user. First sensor signals 42 can comprise respiratory signals, actigraphy signals, or a combination of both. The sleep-onset detection module 32 outputs (i) a non-sleep status in response to not detecting the sleep-onset or a predetermined identified sleep stage, and (ii) an in-sleep status in response to detecting the sleep-onset or another predetermined identified sleep state.

The mask removal detection module 34 is configured to process second sensor signals 44 to detect a status of the mask 18, whether the mask 18 is removed or not. Second sensor signals 44 can comprise airflow sensor signals, pressure sensor signals, in-mask sensor signals, IR sensor signals (e.g., to detect a distance between a PAP mask and a user's forehead), or a combination thereof. The mask removal detection module 34 outputs (i) a mask-off status in response to detecting that the user removed a CPAP mask 18, and (ii) a mask-on status in response to not detecting that the user removed the CPAP mask 18.

The unconscious mask removal detection module 30 is configured to output the indication of one or more removal decisions. A first removal decisions can include (i) a no removal decision in response to (a) the sleep-onset detection module 32 outputting the in-sleep status and the mask removal detection module 34 outputting the mask-on status or (b) the sleep-onset detection module 32 outputting the non-sleep status and the mask removal detection module 34 outputting the mask-on status. A second removal decision can include (ii) an intentional removal decision in response to the sleep-onset detection module 32 outputting the non-sleep status and the mask removal detection module 34 outputting the mask-off status. A third removal decision can include (iii) an unconscious removal decision in response to the sleep-onset detection module 32 outputting the in-sleep status and the mask removal detection module 34 outputting the mask-off status.

Referring still to Figs 2 and 3, controller 22 further comprises a wake-up alert decision module 36 and a personalized alert adjustment module 38. The wake-up alert module 36 is configured to output a decision, as indicated by decision block 48, whether to not activate or activate a wake-up alert based on (i) the output of the unconscious mask removal detection module 30 and (ii) a cost-benefit analysis 50 regarding whether to wake up the user 20 having a positive or negative result based on at least a user treatment goal 52 and user treatment history data 54. In one embodiment, the wake-up alert decision module 36 is configured to compute, via a cost-benefit analysis module 50, a cost-benefit analysis result, as will be discussed further herein.

In one embodiment, a negative cost benefit analysis result can be indicative of an expected cost being higher that an expected benefit, wherein the treatment goal has already been achieved for that night or is above a given threshold of being achieved, and the expected benefit of waking up the user (to have the user put the mask on again) is less than a given low benefit threshold (i.e., marginal). On the other hand, a positive cost benefit analysis result can be indicative of the expected benefit being higher that the expected cost, wherein the treatment goal has not been achieved or is below a given threshold of being achieved and the unconscious mask removal is detected at the beginning of the sleep-onset, and the expected benefit of waking up the user (to have the user put the mask on again) is more than a given high benefit threshold (i.e., large). The wake-up alert decision module 36 outputs the decision, via decision block 48, to (i) not activate the wake-up alert in response to a negative cost-benefit analysis result and the indication of unconscious removal mask-off or (ii) activate the wake-up alert in response to a positive cost-benefit analysis result and the indication of unconscious removal mask-off.

The personalized alert adjustment module 38 can include an adaptive alert module 56 configured to (i) determine alert signal settings for the wake-up alert based on (a) behavioral data 58 of a previous response or previous responses to an alert by the user and (b) historical data 60 of previous alert settings and a response to each previous alert setting by the user. In one embodiment, the alert signal settings can include one or more of a vibration intensity, vibration frequency, or other vibration characteristic (e.g., duty-cycle, modulation, etc. In addition, the personalized alert adjustment module 38, or adaptive alert 56, is configured to (ii) send the determined alert signal settings (e.g., via wired or wireless communication) to the actuator 28 (Fig. 1). The actuator 28 then actuates the wake-up alert in response to the determined wake-up alert signal settings to wake-up the user so that the user 20 can put the unconsciously removed mask on again. The adaptive alert module 38 is operable in response to a decision output of the wake-up alert decision module 36 to activate the wake-up alarm.

In one embodiment, the behavioral data can include data relating to the user's prior response or responses to a given alert that includes one or more of ignoring the given alert, activating a snooze function for the given alert, or other recordable action with respect to the given alert. The historical data can include data relating to a most effective alert setting or settings, compared to a lesser effective alert setting or settings, from a previous night or previous nights to wake up the user. In addition, the personalized alert adjustment module 38, or adaptive alert module 56, can be configured to update one or more of the behavioral data 58 and historical data 60 after each session of wake-up alert activation, for example, based on a mask-on status determined via a query (decision block 62). For example, if the mask is not put back on, then the historical data is updated, via a suitable data update, at 64 that the mask remains off. If the mask is put back on, then the historical data is updated, via a suitable data update, at 62 that the mask is put back on.

In another embodiment, the personalized alert module 38 or the adaptive alert module 56 can be configured to adjust one or more setting of a continuous positive airway pressure (CPAP) system, e.g., system 12 of Fig. 1, wherein the CPAP system can be used in delivering the flow of gas via the mask 18 to the airway of the user respiratory system. The adjusting of one of more settings of the CPAP system can be based on user behavior relating to a threshold number of unconscious mask removal detections. For example, the threshold number can be adapted with respect to a given CPAP implementation and user CPAP treatment history.

With reference to Fig. 3, there is illustrated a schematic representation of the system according to the embodiments of the present disclosure that processes sensor signals to detect unconscious mask removal, determines whether wake-up alert should be activated based on treatment goal and results of detection modules (sleep-onset detection and mask removal detection modules), and sets up personalized alert signals based on behavior and historical data.

In yet another embodiment, a continuous positive airway pressure (CPAP) system is provided for delivering a flow of gas to an airway of a patient respiratory system featuring unconscious mask removal detection with adaptive alerting. The CPAP system comprises a gas flow generator 14, at least one sensor 24, and a controller 22 configured for unconscious mask removal detection and adaptive alerting, as discussed herein. The gas flow generator can be configured to generate the flow of gas, wherein the gas flow generator is further configured to communicate the flow of gas to a patient circuit 16 that includes a mask 18. The at least one sensor can be configured to generate output signals related to at least one characteristic associated with at least one of respiratory, actigraphy, airflow, humidity, pressure, temperature, in-mask sensor signals, or any combination thereof. The controller 22 for unconscious mask removal detection and adaptive alerting can be configured for deciding whether or not to activate the wake-up alarm, and for determining alert signal settings for the wake-up alert and sending the determined alert signal settings to an actuator 28. The actuator 28 can actuate the wake-up alert in response to the determined wake-up alert signal settings to wake-up the user 20 so that the user can put the unconsciously removed mask on again.

As discussed herein, the system can include various modules, including an unconscious mask removal detection module, a wake-up detection module, and a personalized alert adjustment module. In one embodiment, the unconscious mask removal detection module may include two sub-modules, e.g., a sleep detection module and a mask removal module. The sleep detection module detects the sleep-onset/sleep stages based on signals (e.g., respiratory or actigraphy signals) to determine whether the patient is asleep. The mask removal detection module detects the mask removal based on signals (e.g., airflow, pressure sensor signals or in-mask sensor signals, such as IR sensor signals to detect the distance between a mask and forehead).

The wake-up decision module activates a wake-up alert based on the outputs of the unconscious mask removal detection module and cost-benefit analysis result. Pre-defined users' treatment goals (e.g., target CPAP treatment time, target total sleep time, other requirements, etc.) and treatment history (i.e., historical treatment data the previous nights) are processed for cost-benefit analysis. The personalized alert adjustment module adjusts the setting of alert signal (e.g., the intensity or frequency of the alert) based on user behavior (i.e., the response to the alert) and/or historical data (i.e., the most effective setting from the previous nights) and sends signals to the actuator to wake up the user. Optionally, the settings of the CPAP will be adjusted by reflecting the user behavior (e.g., after a certain number of unconscious mask removal detection, the system can decide to adjust air pressure setting). While it could be considered as non-desirable to wake the patient during unconscious mask removal, the consequence for reduced therapy effectiveness due to non-compliance is considered significantly more important. Additionally, as the patient gets used to the therapy, the incidence of unconscious mask removal will reduce and thus cause less waking-up moments.

In one embodiment, a system and method can be implemented which include the following. A CPAP machine (e.g., a Philips DreamStation^{™}) is provided with a treatment goal (e.g., a target time of CPAP therapy) for a night. During the night, the CPAP machine processes data from the CPAP mask usage (e.g., respiratory, airflow, humidity, pressure, temperature sensor signals, etc). The sleep detection module processes sensor signals to detect sleep-onset or identify sleep stages. The output of the sleep detection module can include: (i) Non-Sleep, wherein the sleep detection module outputs non-sleep status when it does not detect sleep-onset or sleep stage is identified as awake time (i.e., before sleep stage 1); and (ii) In-Sleep, wherein sleep detection module outputs in-sleep status when it has detected sleep-onset or depends on the identified sleep stage. Optionally, the sleep stage data (i.e., whether the user is in the deep sleep or light sleep) can be utilized during other decision steps described below.

The mask removal detection module processes sensor signals to detect the status of a mask (whether it is removed or not). The output of the mask removal detection module can include: (i) Mask-Off, wherein the mask removal detection module outputs Mask-Off status when it detects that a user removed a CPAP mask; and (ii) Mask-On, wherein the mask removal detection module outputs Mask-On status when it does not detect that a user removed a CPAP mask.

The final outputs of the unconscious mask removal detection module are as follows: (i) Decision option 1 (None): when (a) sleep detection block outputs In-Sleep AND the mask removal detection module outputs Mask-On; or (b) sleep detection block outputs Non-Sleep AND the mask removal detection module outputs Mask-On; (ii) Decision option 2 (IntentionalRemoval): when sleep detection module outputs Non-Sleep result and mask removal detection module outputs Mask-Off result; and (iii) Decision option 3 (UnconsciousRemoval): when the sleep detection module outputs In-Sleep result, mask removal detection module outputs Mask-On result.

When the wake-up alert decision module takes decision option 3 (UnconsciousRemoval) from the unconscious mask removal module, it computes the expected cost and benefit of each decision option. To determine whether it is worth waking up the user, user's treatment goal and treatment history data (i.e., usage data from previous nights) are used. The final outputs of the wake-up decision module are as follows: Decision option 1 (NoActivation): when cost-benefit analysis result is negative (i.e., the expected cost is higher than the expected benefit). For example, the treatment goal has been already achieved/is almost achieved and the expected benefit of waking up the user is marginal. Decision option 2 (Activation): when cost-benefit analysis result is positive (i.e., the expected benefit is higher than the expected cost). For example, the unconscious mask removal is detected at the beginning of the sleep and the expected benefit of waking up the user is large.

The wake-up alert will be activated only when the wake-up decision module outputs decision option 2 (Activation). Upon activation, the adaptive alert module determines the alert setting (i.e., vibration intensity, frequency, etc) based on user behavior and historical data. Behavioral data may include a user response to the alert (e.g., ignoring an alert, hitting a snooze button, etc). Historical data may include previous alert settings and user response to each respective alert setting. The adaptive alert module sends a signal to an actuator (e.g., smartwatch, a wearable device with vibration function, such as Philips NightBalance, etc, mobile device, alarm clock, PAP base station) to wake up the user so that the user can put on the mask again. Optionally, the settings of the CPAP will be adjusted by reflecting the user behavior (e.g., after a certain number of unconscious mask removal detection (threshold), the system can decide to adjust air pressure setting. The threshold value can be defined or adapted to the context. Lastly, behavioral and historical data may be updated after each session of alert activation.

According to one embodiment, a method for unconscious mask removal detection and adaptive alerting in a system for delivering a flow of gas via a mask worn by a user to an airway of a user respiratory system can comprise detecting unconscious mask removal, deciding whether to activate or not a wake-up alert, and determining alert signal settings. Detecting the unconscious mask removal can include detecting, via the unconscious mask removal module 30, and outputting an indication of unconscious removal mask-off or other than unconscious removal mask-off. The indication of other than unconscious removal mask-off may include an indication of mask-on or intentional removal mask-off. Deciding whether to activate or not the wake-up alert can include deciding, via the wake-up alert decision module 36, whether to not activate or activate a wake-up alert based on (i) the output of the unconscious mask removal detection module 30 and (ii) a cost-benefit analysis 50 regarding whether to wake up the user having a positive or negative result based on at least a user treatment goal and user treatment history data. Lastly, determining alert signal settings can include determining, via the adaptive alert module 56, alert signal settings for the wake-up alert based on (a) behavioral data of a previous response or previous responses to an alert by the user and (b) historical data of previous alert settings and a response to each previous alert setting by the user. The method further includes sending, via the adaptive alert module 56, the determined alert signal settings to the actuator 28 for the actuator to actuate the wake-up alert in response to the determined wake-up alert signal settings to wake-up the user so that the user can put the unconsciously removed mask on again. The adaptive alert module 56 is operable in response to a decision output of the wake-up alert decision module 36 to activate the wake-up alarm.

In another embodiment, the method can include wherein detecting, via the unconscious mask removal detection module 30, further includes detecting, via a sleep-onset detection module 32 and a mask removal detection module 34. The sleep-onset detection module 32 is configured to process first sensor signals 42 to detect a sleep-onset or identify sleep stages of the user. The mask removal detection module 34 is configured to process second sensor signals 44 to detect a status of a mask 18, whether the mask is removed or not. The first sensor signals 42 can comprise respiratory signals, actigraphy signals, or a combination of both. The second sensor signals 44 can comprise airflow sensor signals, pressure sensor signals, in-mask sensor signals, IR sensor signals (e.g., to detect a distance between a PAP mask and a user's forehead), or a combination thereof.

According to yet another embodiment, the method includes wherein deciding, via the wake-up alert decision module, further comprises computing the cost-benefit analysis result. A negative cost benefit analysis result is indicative of an expected cost being higher that an expected benefit, wherein the treatment goal has already been achieved or is above a given threshold of being achieved, and the expected benefit of waking up the user (to have the user put the mask on again) is less than a given low benefit threshold (i.e., marginal). A positive cost benefit analysis result is indicative of the expected benefit being higher that the expected cost, wherein the treatment goal has not been achieved or is below the given threshold of being achieved and the unconscious mask removal is detected at the beginning of the sleep-onset, and the expected benefit of waking up the user (to have the user put the mask on again) is more than a given high benefit threshold (i.e., large).

Although only a few exemplary embodiments have been described in detail above, those skilled in the art will readily appreciate that many modifications are possible in the exemplary embodiments without materially departing from the novel teachings and advantages of the embodiments of the present disclosure. For example, the embodiments of the present disclosure can be advantageously used in systems designed for patients with positional OSA to guide the patients to adjust their sleep postures by providing light vibration. Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A system (10) for unconscious mask removal detection and adaptive alerting in delivering a flow of gas via a mask (18) worn by the user (20) to an airway of a user respiratory system, the system comprising:
an unconscious mask removal detection module (30) configured to detect an unconscious mask removal, wherein the mask removal detection module outputs an indication of unconscious removal mask-off or other than unconscious removal mask-off;
**characterized in that** the system comprises:
a wake-up alert decision module (36) configured to output a decision whether to not activate or activate a wake-up alert based on (i) the output of the unconscious mask removal detection module and (ii) a cost-benefit analysis regarding whether to wake up the user having a positive or negative result based on at least a treatment goal and treatment history data; and
an adaptive alert module (38) configured to (i) determine alert settings for the wake-up alert based on (a) behavioral data of a previous response or previous responses to an alert by the user and (b) historical data of previous alert settings and a response to each previous alert setting by the user, and (ii) send the determined alert settings to an actuator for the actuator to actuate the wake-up alert in response to the determined wake-up alert settings to wake-up the user so that the user can put the unconsciously removed mask on again, wherein the adaptive alert module is operable in response to a decision output of the wake-up alert decision module to activate the wake-up alarm.

2. The system (10) according to claim 1, wherein the unconscious mask removal detection module (30) further includes a sleep-onset detection module (32) and a mask removal detection module (34), wherein the sleep-onset detection module (32) is configured to process first sensor signals to detect a sleep-onset or identify sleep stages of the user, and wherein the mask removal detection module (34) is configured to process second sensor signals to detect a status of the mask, whether the mask is removed or not.

3. The system (10) according to claim 2, wherein the first sensor signals (42) comprise respiratory signals, actigraphy signals, or a combination of both, and wherein the second sensor signals (44) comprise airflow sensor signals, pressure sensor signals, in-mask sensor signals, IR sensor signals, or a combination thereof.

4. The system (10) according to claim 2, wherein the sleep-onset detection module (32) outputs (i) a non-sleep status in response to not detecting the sleep-onset or a predetermined identified sleep stage, and (ii) an in-sleep status in response to detecting the sleep-onset or another predetermined identified sleep state.

5. The system (10) according to claim 2, wherein the mask removal detection module (34) outputs (i) a mask-off status in response to detecting that the user removed a CPAP mask, and (ii) a mask-on status in response to not detecting that the user removed the CPAP mask.

6. The system (10) according to claim 2, wherein the unconscious mask removal detection module (34) outputs the indication of:
(i) a no removal decision in response to (a) the sleep-onset detection module (32) outputting the in-sleep status and the mask removal detection module (34) outputting the mask-on status or (b) the sleep-onset detection module (32) outputting the non-sleep status and the mask removal detection module (34) outputting the mask-on status,
(ii) an intentional removal decision in response to the sleep-onset detection module (32) outputting the non-sleep status and the mask removal detection module (34) outputting the mask-off status, and
(iii) an unconscious removal decision in response to the sleep-onset detection module (32) outputting the in-sleep status and the mask removal detection module (34) outputting the mask-off status.

7. The system (10) according to claim 1, wherein the wake-up alert decision module (36) is further configured to compute the cost-benefit analysis result, wherein the negative cost benefit analysis result is indicative of an expected cost being higher that an expected benefit, wherein the treatment goal has already been achieved or is above a given threshold of being achieved, and the expected benefit of waking up the user is less than a given low benefit threshold, wherein the positive cost benefit analysis result is indicative of the expected benefit being higher that the expected cost, wherein the treatment goal has not been achieved or is below a given threshold of being achieved and the unconscious mask removal is detected at the beginning of the sleep-onset, and the expected benefit of waking up the user is more than a given high benefit threshold, and wherein the wake-up alert decision module (36) outputs the decision to (i) not activate in response to a negative cost-benefit analysis result and the indication of unconscious removal mask-off or (ii) activate in response to a positive cost-benefit analysis result and the indication of unconscious removal mask-off.

8. The system (10) according to claim 1, wherein the behavioral data (58) includes data relating to the user's prior response or responses to a given alert that includes one or more of ignoring the given alert, activating a snooze function for the given alert, or other recordable action with respect to the given alert, and wherein the historical data (60) includes data relating to a most effective alert setting or settings, compared to a lesser effective alert setting or settings, from a previous night or previous nights to wake up the user.

9. The system (10) according to claim 1, wherein the adaptive alert module (38) is further configured to update one or more of the behavioral data and historical data after each session of wake-up alert activation.

10. The system (10) according to claim 1, wherein the actuator (28) includes one or more of a smartwatch, a wearable device with a vibration function, a mobile device, an alarm clock, a positive airway pressure (PAP) base station, or other vibration device.

11. The system (10) according to claim 1, wherein the adaptive alert module (38) is further configured to adjust one or more setting of a continuous positive airway pressure (CPAP) system, used in delivering the flow of gas via the mask to the airway of the user respiratory system, based on user behavior relating to a threshold number of unconscious mask removal detections, wherein the threshold number is adapted with respect to a given CPAP implementation and user CPAP treatment history.

12. A continuous positive airway pressure (CPAP) system (12) for delivering a flow of gas to an airway of a patient respiratory system featuring unconscious mask removal detection with adaptive alerting, the system comprising:
a gas flow generator (14) configured to generate the flow of gas, wherein the gas flow generator is further configured to communicate the flow of gas to a patient circuit (16) that includes a mask (18);
at least one sensor (24) configured to generate output signals related to at least one characteristic associated with at least one of respiratory, actigraphy, airflow, humidity, pressure, temperature, in-mask sensor signals, or any combination thereof; and
the system (10) for unconscious mask removal detection and adaptive alerting according to claim 1, for deciding whether or not to activate the wake-up alarm, and for determining alert settings for the wake-up alert and sending the determined alert settings to an actuator (28) for the actuator to actuate the wake-up alert in response to the determined wake-up alert settings to wake-up the user so that the user can put the unconsciously removed mask on again.

13. A method for unconscious mask removal detection and adaptive alerting in a system for delivering a flow of gas via a mask worn by a user to an airway of a user respiratory system, the method comprising:
detecting, via an unconscious mask removal module, an unconscious mask removal, wherein the mask removal detection module outputs an indication of unconscious removal mask-off or other than unconscious removal mask-off;
**characterized in that** the method comprises:
deciding, via a wake-up alert decision module, whether to not activate or activate a wake-up alert based on (i) the output of the unconscious mask removal detection module and (ii) a cost-benefit analysis regarding whether to wake up the user having a positive or negative result based on at least a treatment goal and treatment history data; and
determining, via an adaptive alert module, alert settings for the wake-up alert based on (a) behavioral data of a previous response or previous responses to an alert by the user and (b) historical data of previous alert settings and a response to each previous alert setting by the user, and sending, via the adaptive alert module, the determined alert settings to an actuator for the actuator to actuate the wake-up alert in response to the determined wake-up alert settings to wake-up the user so that the user can put the unconsciously removed mask on again, wherein the adaptive alert module is operable in response to a decision output of the wake-up alert decision module to activate the wake-up alarm.

14. The method according to claim 13, wherein detecting, via the unconscious mask removal detection module, further includes detecting, via a sleep-onset detection module and a mask removal detection module, wherein the sleep-onset detection module is configured to process first sensor signals to detect a sleep-onset or identify sleep stages of the user, and wherein the mask removal detection module is configured to process second sensor signals to detect a status of a mask, whether the mask is removed or not, wherein the first sensor signals comprise respiratory signals, actigraphy signals, or a combination of both, and wherein the second sensor signals comprise airflow sensor signals, pressure sensor signals, in-mask sensor signals, IR sensor signals, or a combination thereof.

15. The method according to claim 13, wherein deciding, via the wake-up alert decision module, further comprises computing the cost-benefit analysis result, wherein the negative cost benefit analysis result is indicative of an expected cost being higher that an expected benefit, wherein the treatment goal has already been achieved or is above a given threshold of being achieved, and the expected benefit of waking up the user is less than a given low benefit threshold, and wherein the positive cost benefit analysis result is indicative of the expected benefit being higher that the expected cost, wherein the treatment goal has not been achieved or is below the given threshold of being achieved and the unconscious mask removal is detected at the beginning of the sleep-onset, and the expected benefit of waking up the user is more than a given high benefit threshold.
